Europäisches Patentamt

(19) European Patent Office

Office européen des brevets

(11) Publication number: **0 037 223**
**B1**

(12) **EUROPEAN PATENT SPECIFICATION**

(45) Date of publication of patent specification: **13.07.83**   (51) Int. Cl.³: **A 61 N  1/04**

(21) Application number: **81301206.9**

(22) Date of filing: **20.03.81**

(54) A body implantable lead having a ring electrode, and a process for making same.

(30) Priority: **21.03.80 US  133371**

(43) Date of publication of application:
**07.10.81 Bulletin 81/40**

(45) Publication of the grant of the patent:
**13.07.83 Bulletin 83/28**

(84) Designated Contracting States:
**DE FR GB NL**

(56) References cited:
**EP - A - 0 015 229**
**FR - A - 2 299 873**
**GB - A - 1 124 143**
**US - A - 3 350 500**
**US - A - 3 428 739**
**US - A - 3 683 932**
**US - A - 3 769 984**
**US - A - 3 871 382**
**US - A - 4 030 508**
**US - A - 4 214 804**

(73) Proprietor: **MEDTRONIC, INC.**
**3055 Old Highway Eight P.O. Box 1453**
**Minneapolis Minnesota 55440 (US)**

(72) Inventor: **Ufford, Keith A.**
**17302 68th Avenue North**
**Maple Grove Minnesota 55369 (US)**
Inventor: **O'Neill, Edward G.**
**1840 Clarence**
**St. Paul Minnesota 55109 (US)**

(74) Representative: **Boyes, Kenneth Aubrey et al,**
**Frank B. Dehn & Co. Imperial House 15-19**
**Kingsway**
**London WC2B 6UZ (GB)**

Courier Press, Leamington Spa, England

# A body implantable lead having a ring electrode and a process for making same

The present invention relates to a body implantable lead for use as a surgical electrical applicator having a ring electrode and a method for making same, and more particularly though not exclusively to a bipolar pacing lead having a ring electrode and process of making same.

Existing types of bipolar pacing leads have sometimes presented an uneven surface at the junction of the ring electrode and the insulation of the insulated coiled conductor of the bipolar pacing lead. The tensile strength of the ring electrode at the interface of the insulated coiled conductor has been considerably less than desirable when the coiled conductor has been welded to the ring electrode.

United States Patent No. 3,871,382 discloses a catheter in which an annular electrode is welded to an inner shell by means of two annular welds. The assembly thus formed is fitted over a coiled conductor and the inner shell is welded to the coiled conductor at several annular areas. Finally, an insulating sleeve is pulled up to a point near the proximal end of the electrode. Electrical contact between the electrode and the coiled conductor is thus dependent on the quality of the welds between the electrode and the inner shell and between the inner shell and the coiled conductor, the latter welds having to be made after assembly of the device.

United States Patent No. 4,030,508 discloses a catheter comprising a distal electrode. The electrode includes a sleeve which surrounds and makes electrical contact with a coiled conductor. The electrode is in turn surrounded by a silicone rubber flange which also surrounds the coil insulation.

According to the invention there is provided a body implantable lead including a coiled conductor having a terminal pin at the proximal end thereof; insulation over said coiled conductor; and a ring electrode disposed over the distal end of said coiled conductor and a swaging core disposed inside the distal end of said coiled conductor, said ring electrode having been pulled over said distal end of said coiled conductor to said swaging core whereby said coiled conductor is swaged between said electrodes and said swaging core and electrical continuity is provided between said coiled conductor and said ring electrode.

The present invention provides a ring electrode which is swaged to the coiled conductor, e.g. the outer conductor of a bipolar pacing lead. The lead thus has enhanced tensile strength about the outer coiled conductor. In addition, the lead offers economic advantages in being easily and readily produceable with little change for manufacturing defects providing for consistent manufacturing assembly. The construction at the same time enables a smooth transition between the insulation and the ring electrode to be provided since the mechanical and electrical connection between the ring electrode and electrical conductor is within the ring electrode: thus the insulation of the coiled conductor can abut up to an end of the ring electrode.

According to one embodiment of the present invention, there is provided a ring electrode on an outer coiled conductor of a bipolar pacing lead comprising an outer coiled conductor, insulation over the outer coiled conductor, a swaging core positioned internally into the distal end of the outer coiled conductor, a ring electrode, positioned externally over the distal end of the outer coiled conductor, and insulation of the outer coiled conductor abutting up to the proximal end of the ring electrode; and insulation between a pacing tip at the distal end of the bipolar lead and the distal end of the ring electrode abutting up the distal end of the ring electrode; wherein the outer coiled conductor is swaged between the ring electrode and the swaging core.

In a preferred embodiment of the invention, the lead comprises a coiled conductor disposed inside the first mentioned coiled conductor; insulation over said inner coiled conductor; the distal end of said coiled conductor being connected to a tip electrode and the proximal end being connected to a terminal pin; thereby forming a bipolar lead with said first mentioned coiled conductor being the outer coiled conductor.

A further significant aspect and feature of the present invention, particularly as applied to a bipolar pacing lead, is that in the area surrounding the ring electrode the lead can be flexible.

Flexibility and a smooth transition between insulation and ring electrode enables a pacing lead to be provided which is easy to pass around curves, especially in veins.

A preferred embodiment of the present invention provides a pacing lead including a ring electrode on a bipolar pacing lead and having enhanced tensile strength about the outer coiled conductor at the ring electrode. The coiled conductor is swaged between the ring electrode and the swaging core thereby providing a smooth surface on the outer insulated surface of the pacing lead. The smooth outer surface is particularly desirable and provides for easy passage of the bipolar pacing lead through veins of the patient's body and lead insertion devices, and further minimizes thrombus formation.

Other objects and many of the attendant advantages of this invention will be readily appreciated as the same becomes better understood by reference to the following detailed description of a preferred embodiment of the invention with reference to the accompanying drawing, in which like reference numerals designate like parts through the FIGURES thereof and wherein:

Fig. 1 illustrates a plan view of a bipolar pacing lead having a ring electrode according to the present invention; and

Fig. 2 illustrates a cross-sectional view taken along line 2—2 of Fig. 1.

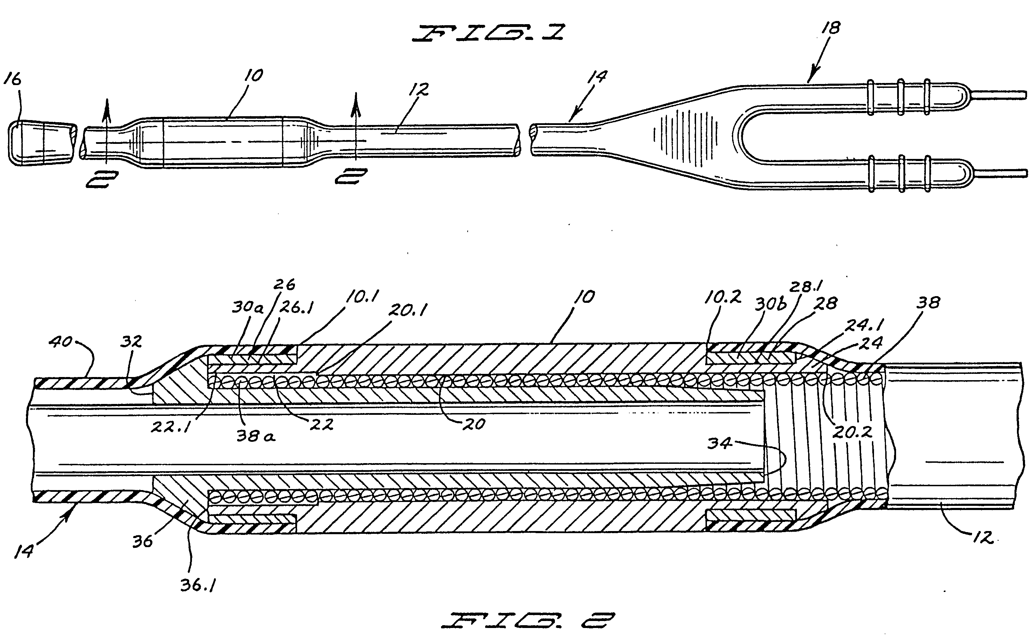

Fig. 1, shows a bipolar pacing lead 14 having a ring electrode 10, insulation 12 and also a pacing tip electrode 16 at a distal end and terminal pins 18 at the proximal end of the pacing lead 14.

Fig. 2 shows the ring electrode 10 on the pacing lead 14 adjacent the distal end where the outer diameter of the ring electrode 10 is slightly larger than the outer diameter of the insulation 12 of the pacing lead 14. The ring electrode 10 can be platinum, platinum-iridium, or other like material. The insulation 12 of the pacing lead 14 surrounds outer coiled conductor 38 which can be multi-filar such as quadrafilar nickel alloy coiled conductor commonly referred to as MP35N by way of example and for purposes of illustration only, and which can be manufactured under the Drawn-Brazed-Stranded (DBS) process. The insulation 12 can be urethane, polyether urethane elastomer or other like material as now used for insulation such a coiled conductor 38. The pacing inner coiled conductor is not illustrated for purposes of clarity in the drawing and for purposes of brevity in the specification.

The ring electrode 10 includes an outer diameter or cylindrical surface which provides for pacing from distal outer end 10.1 to proximal outer end 10.2, an inner diameter 20 from distal inner end 20.1 to proximal inner end 20.2, and a slightly elongated large inner coil relief channel or rebate 22 which provides for coil relief from distal inner end 22.1 of channel 22 to distal inner end 20.1 of internal diameter 20. A hook 24 is provided adjacent the proximal inner end 20.2. External longitudinal circular channel or rebate 26 is formed by a smaller outer diameter 26.1 and external channel 28 is formed by a small diameter outer diameter 28.1 and a hook 24 is at one end of the ring electrode 10 providing for bands of tubing in the channels as now described in detail. Distal and proximal bands of tubing 30a and 30b respectively such as urethane or other like material and which can be analogized to a rubber band, snap in and engage within the external channels 26 and 28 respectively for receiving insulation as later described in detail. A swaging core 32 includes a slightly tapered proximal end 34 and a distal hook end 36 reciprocal in structure to the hook end 24 of the ring electrode 10. The swaging core 32 has an outer diameter less than the inner diameter of the coiled conductor 38 and of the same or like material as the ring electrode 10.

The process for securing the ring electrode 10 to the distal end of an outer coaxial coiled conductor 38 comprises the steps of positioning urethane bands 30a and 30b in the channels 26 and 28 respectively, sliding the ring electrode 10 over the coiled conductor 38 adjacent the distal end of the outer coiled conductor 38, inserting the swaging core 32 having the tapered end 34 so that a hook edge 36.1 abuts up against the end winding of the coiled conductor 38 and pulling said ring electrode 10 to said swaging core 32 to force the ring electrode 10 distal end to the hook edge 36.1 of the swaging core 32 so swaging the coiled conductor 38 between the ring electrode 10 and the swaging core 32, possibly with the aid of an internal mandrel having substantially the same inner diameter as swaging core 32. The coil relief channel 22 takes up any extra slack of the distal end of the outer coiled conductor 38. Insulation 12 is then threaded over the outer coiled conductor 38 and bonded to band 30b. Subsequent steps can include the threading of a coaxial inner insulated coiled conductor not illustrated in the figures for purposes of clarity in the drawing through the outer coiled conductor 38, through the swaging core 32, and bonding of insulation 40 from the pacing tip electrode 16 over the band 30a to abut up against the distal outer end 10.1. The ends 10.1 and 10.2 of the ring electrode 10 having the insulation over the bands 30a and 30b in the channels 26 and 28 assume substantially the same outer diameter as the outer diameter of the ring electrode 10 which is slightly larger than the diameter of the insulation 12. The hooks 24 and 36 and hook edges 24.1 and 36.1 serve to retain the insulation 12 and 40 over the pacing tip electrode 16 in position in addition to providing outer ends for the channels 28 and 26 respectively and abutment between the ring electrode 10 and the swaging core 32. A plurality of tines not illustrated in the figure can extend from the insulation 40 about the pacing tip electrode 16.

The mechanical joint resulting by the swaging of the coil conductor 38 between elements 10 and 32 can be considered as a mechanical weld which provides mechanical and electrical connection of the ring electrode 10 to the coiled conductor 38 about the swaging core 32.

The resultant pacing lead 14 includes a ring electrode 10 which provides a smooth outer surface for easy lead passage through veins and a lead introducer if utilized. The swaging of ring electrode 10 provides flexibility in the area of the ring electrode 10 on the pacing lead 14, which aids easy passage of the pacing lead 14 around curves, and the tensile strength at the ring electrode 10 is substantially equal that of the insulation 12 utilized over the coiled conductor 38.

Alternative embodiments of the ring electrode for a pacing lead are forseeable in light of the above specification. One embodiment is a body-implantable lead for pacing, sensing, stimulating or like medical applications and includes electrode applicator structure at the end of the lead where the electrode application includes the swaging core having a rounded forward portion, the distal end of the conductor, and a cylindrical tubular conductor member in lieu of the ring electrode structure and including only one circumferential channel at the proximal end. Ring electrode can include the structure of a cylindrical tubular conductive member having an outer cylindrical surface. Also, the ring electrode can include only one channel for a band of insulation, either distal or proximal, or in the alternative, one of the bands can be used for bonding insulation to one end of the electrode.

Various modifications can be made to the ring electrode 10 of the pacing lead of the present invention without departing from the apparent scope thereof.

## Claims

1. A body implantable lead including a coiled conductor (38) having a terminal pin (18) at the proximal end thereof; insulation (12) over said coiled conductor; and a ring electrode (10) disposed over the distal end (38a) of said coiled conductor, characterised in that a swaging core (32) is disposed inside the distal end of said coiled conductor, said ring electrode having been pulled over said distal end of said coiled conductor to said swaging core whereby said coiled conductor is swaged between said ring electrode and said swaging core and electrical continuity is provided between said coiled conductor and said ring electrode.

2. A body implantable lead as claimed in claim 1 wherein the insulation abuts the proximal end (10.2) of an outer cylindrical surface of the ring electrode, whereby a smooth surface is provided at the abutment of the ring electrode and the insulation.

3. A body implantable lead as claimed in claim 1 or 2 comprising an annular channel (28) in the proximal end of said ring electrode and a proximal end band of insulation (30b) disposed in said channel; said insulation of said coiled conductor being bonded to said proximal band of insulation.

4. A body implantable lead as claimed in claim 3 comprising an annular hook (24) including a hook edge (24.1) on the proximal end of said ring electrode; said hook edge of said hook engaging said proximal band.

5. A body implantable lead as claimed in any preceding claim comprising a coil relief rebate (22) at the internal distal end of said ring electrode, said rebate having a diameter slightly larger than the internal diameter of said ring electrode whereby said coil relief rebate accepts said distal end of said coiled conductor.

6. A body implantable lead as claimed in any preceding claim wherein said swaging core comprises a cylindrical member including a slightly tapered proximal end (34).

7. A body implantable lead as claimed in any preceding claim wherein an annular rebate (26) is provided at the distal end of said ring electrode; and a distal band of insulation (30a) is disposed in said rebate; distal end insulation extending from the distal end of the ring electrode towards the distal end of the lead, being bonded to said distal band of insulation.

8. A body implantable lead as claimed in claim 7 wherein said swaging core comprises an annular hook (36) at its distal end; said hooking acting in combination with said ring electrode thereby forming a channel containing said distal band of insulation.

9. A body implantable lead as claimed in any preceding claim wherein said coiled conductor is multifilar.

10. A body implantable lead as claimed in any preceding claim wherein said insulation is urethane.

11. A body implantable lead as claimed in any one of claims 1 to 9 wherein said insulation is polyether urethane elastomer.

12. A body implantable lead as claimed in any preceding claim comprising a coiled conductor disposed inside the first mentioned coiled conductor; insulation over said inner coiled conductor; the distal end of said inner coiled conductor being connected to a tip electrode (16) and the proximal end being connected to a terminal pin; thereby forming a bipolar lead with said first mentioned coiled conductor being the outer coiled conductor.

13. A process of swaging a ring electrode to a coiled conductor (38) of a body implantable lead comprising the steps of:

a. positioning the ring electrode (10) including an annular inner coil relief channel (22) at a distal end of said ring electrode, an annular hook (24) at the proximal end of said ring electrode, and two elongate annular channels (26, 28) on the external diameter of the distal and proximal ends of said ring electrode, adjacent the distal end of said coiled conductor;

b. engaging distal and proximal bands of insulation (30a, 30b) in said elongate channels;

c. inserting an annular swaging core (32) including a proximal tapered end (34) and an annular distal hooked end (36) into said distal end of the coiled conductor; and

d. pulling said ring electrode to said swaging core, thereby swaging said coil conductor therebetween and bonding insulation (12) of said coiled conductor to said proximal band and said distal band.

14. The process of claim 13 wherein said step of bonding insulation to said bands over the ring electrode yields insulation over said bands where said insulation overlapping said ends of said ring electrode is substantially the same outer diameter as said ring electrode.

**Patentansprüche**

1. Eine in den Körper implantierbare Leitung mit einem gewendelten Leiter (38), der an seinem proximalen Ende einen Anschlußstift (18) aufweist; einer Isolation (12) über dem gewendelten Leiter; und einer über dem distalen Ende (38a) des gewendelten Leiters angeordneten Ringelektrode (10), dadurch gekennzeichnet, daß in dem distalen Ende des gewendelten Leiters ein Preßkern (32) angeordnet und die Ringelektrode über das distale Ende des gewendelten Leiters auf den Preßkern gezogen ist, wodurch der gewendelte Leiter zwischen der Ringelektrode und dem Preßkern verpreßt und für eine elektrische Verbindung zwischen dem gewendelten Leiter und der Ringelektrode gesorgt ist.

2. Eine in den Körper implantierbare Leitung nach Anspruch 1, dadurch gekennzeichnet, daß die Isolation an dem proximalen Ende (10.2) einer zylindrischen Außenfläche der Ringelektrode anliegt, wodurch an' der Treffstelle von Ringelektrode und Isolation eine glatte Oberfläche ausgebildet ist.

3. Eine in den Körper implantierbare Leitung nach Anspruch 1 oder 2, gekennzeichnet durch einen Ringkanal (28) in dem proximalen Ende der Ringelektrode und ein in diesem Kanal sitzendes, dem proximalen Ende zugeordnetes Isolierband (30b), wobei die Isolation des gewendelten Leiters mit dem proximalen Isolierband verbunden ist.

4. Eine in den Körper implantierbare Leitung nach Anspruch 3, gekennzeichnet durch einen ringförmigen Haken (24) mit einem Hakenrand (24.1) an dem proximalen Ende der Ringelektrode, wobei der Hakenrand des Hakens mit dem proximalen Band in Eingriff steht.

5. Eine in den Körper implantierbare Leitung nach einem der vorhergehenden Ansprüche, gekennzeichnet durch einen an dem inneren distalen Ende der Ringelektrode vorgesehenen Wendelentlastungsfalz (22), der einen Durchmesser aufweist, der etwas größer als der Innendurchmesser der Ringelektrode ist, wobei der Wendelentlastungsfalz das distale Ende des gewendelten Leiters aufnimmt.

6. Eine in den Körper implantierbare Leitung nach einem der vorhergehenden Ansprüche, dadurch gekennzeichnet, daß der Preßkern ein zylindrisches Bauteil mit einem leicht verjüngten proximalen Ende (34) aufweist.

7. Eine in den Körper implantierbare Leitung nach einem der vorhergehenden Ansprüche, dadurch gekennzeichnet, daß an dem distalen Ende der Ringelektrode ein ringförmiger Falz (26) vorgesehen ist und daß ein distales Isolierband (30a) in dem Falz angeordnet ist, wobei die Isolation am distalen Ende, die sich von dem distalen Ende der Ringelektrode in Richtung auf das distale Ende der Leitung erstreckt, mit dem distalen Isolierband verbunden ist.

8. Eine in den Körper implantierbare Leitung nach Anspruch 7, dadurch gekennzeichnet, daß der Preßkern an seinem distalen Ende einen ringförmigen Haken (36) aufweist, der zusammen mit der Ringelektrode einen das distale Isolierband aufnehmenden Kanal bildet.

9. Eine in den Körper implantierbare Leitung nach einem der vorhergehenden Ansprüche, dadurch gekennzeichnet, daß der gewendelte Leiter mehradrig ist.

10. Eine in den Körper implantierbare Leitung nach einem der vorhergehenden Ansprüche, dadurch gekennzeichnet, daß die Isolation aus Urethan besteht.

11. Eine in den Körper implantierbare Leitung nach einem der Ansprüche 1 bis 9, dadurch gekennzeichnet, daß die Isolation aus Polyätherurethanelastomer besteht.

12. Eine in den Körper implantierbare Leitung nach einem der vorhergehenden Ansprüche, gekennzeichnet durch einen gewendelten Leiter, der innerhalb des zuerst genannten gewendelten Leiters angeordnet ist und eine Isolation über dem inneren gewendelten Leiter, wobei das distale Ende des inneren gewendelten Leiters mit einer Spitzenelektrode (16) und das proximale Ende mit einem Anschlußstift unter Bildung einer bipolaren Leitung verbunden sind, wobei der zuerst genannte gewendelte Leiter der äußere gewendelte Leiter ist.

13. Verfahren zum Verpressen einer Ringelektrode mit einem gewendelten Leiter (38) einer in den Körper implantierbaren Leitung, dadurch gekennzeichnet daß:

(a) die Ringelektrode (10), die einen ringförmigen inneren Wendelentlastungskanal (22) an einem distalen Ende der Ringelektrode, einen ringförmigen Haken (24) an dem proximalen Ende der Ringelektrode und zwei langgestreckte ringförmige Kanäle (26, 28) an der Außenseite der distalen und proximalen Enden der Ringelektrode aufweist, benachbart dem distalen Ende des gewendelten Leiters angeordnet wird;

(b) distale und proximale Isolierbänder (30a, 30b) in die langgestreckten Kanäle eingebracht werden;

(c) ein ringförmiger Preßkern (32) mit einem verjüngten proximalen Ende (34) und einem ringhakenförmigen distalen Ende (36) in das distale Ende des gewendelten Leiters eingesetzt wird; und

(d) die Ringelektrode auf den Preßkern gezogen wird, wodurch der gewendelte Leiter zwischen diesen Bauteilen verpreßt wird und die Isolation (12) des gewendelten Leiters mit dem proximalen Band und dem distalen Band verbunden wird.

14. Verfahren nach Anspruch 13, dadurch gekennzeichnet, daß die Verbindung der Isolation mit den Bändern über der Ringelektrode zu einer über den Bändern liegenden Isolation führt, bei welcher die die Enden der Ringelektrode überlappende Isolation im wesentlichen den gleichen Außendurchmesser wie die Ringelektrode hat.

## Revendications

1. Un conducteur implantable dans le corps comprenant un élément conducteur enroulé (38) ayant une broche de borne (18) à son extrémité proximale; un isolant (12) sur cet élément conducteur enroulé; et une électrode annulaire (10) disposée sur l'extrémité distale (38a) de l'élément conducteur enroulé, caractérisé en ce qu'un noyau d'emmanchement (32) est disposé à l'intérieur de l'extrémité distale de l'élémen conducteur enroulé, l'électrode annulaire ayant été tirée sur l'extrémite distale de l'élément conducteur enroulé, sur le noyau d'emmanchement, grâce à quoi l'élément conducteur enroulé est emmanché entre l'électrode annulaire et le noyau d'emmanchement et la continuité électrique est établie entre l'élément conducteur enroulé et l'électrode annulaire.

2. Un conducteur implantable dans le corps tel que revendiqué dans la revendication 1, dans lequel l'isolant vient en contact avec l'extrémité proximale (10.2) d'une surface cylindrique extérieure de l'électrode annulaire, grâce à quoi une surface lisse est établie à la jonction entre l'électrode annulaire et l'isolant.

3. Un conducteur implantable dans le corps tel que revendiqué dans la revendication 1 ou 2, comprenant un chambrage annulaire (28) dans l'extrémité proximale de l'électrode annulaire et une bande d'isolant d'extrémité proximale (30b) disposée dans ce chambrage; et l'isolant de l'élément conducteur enroulé est fixé à la bande proximale d'isolant.

4. Un conducteur implantable dans le corps tel que revendiqué dans la revendication 3, comprenant un crochet annulaire (24) qui comporte une face de crochet (24.1) sur l'extrémité proximale de l'électrode annulaire; et cette face de crochet du crochet vient en contact avec la bande proximale.

5. Un conducteur implantable dans le corps tel que revendiqué dans n'importe quelle revendication précédente, comprenant une feuillure de dégagement (22) pour l'élément conducteur enroulé, à l'extrémité distale interne de l'électrode annulaire, cette feuillure ayant un diamètre légèrement supérieur au diamètre interne de l'électrode annulaire, grâce à quoi la feuillure de dégagement pour l'élément conducteur enroulé reçoit l'extrémité distale de l'élément conducteur enroulé.

6. Un conducteur implantable dans le corps tel que revendiqué dans n'importe quelle revendication précédente, dans lequel le noyau d'emmanchement comprend une pièce cylindrique ayant une extrémité proximale (34) légèrement effilée.

7. Un conducteur implantable dans le corps tel que revendiqué dans n'importe quelle revendication précédente, dans lequel une feuillure annulaire (26) est formée à l'extrémité distale de l'électrode annulaire; et une bande d'isolant distal (30a) est disposée dans cette feuillure; l'isolant d'extrémité distale s'étendant depuis l'extrémité distale de l'électrode annulaire vers l'extrémité distale du conducteur, qui est fixée à cette bande d'isolant distal.

8. Un conducteur implantable dans le corps tel que revendiqué dans la revendication 7, caractérisé en ce que le noyau d'emmanchement comprend un crochet annulaire (36) à son extrémité distale; et ce crochet agit en combinaison avec l'électrode annulaire de façon à former un chambrage contenant la bande d'isolant distal.

9. Un conducteur implantable dans le corps tel que revendiqué dans n'importe quelle revendication précédente, dans lequel l'élément conducteur enroulé est multifilaire.

10. Un conducteur implantable dans le corps tel que revendiqué dans n'importe quelle revendication précédente, dans lequel l'isolant consiste en uréthane.

11. Un conducteur implantable dans le corps tel que revendiqué dans n'importe laquelle des revendications 1 à 9, caractérisé en ce que l'isolant consiste en un élastomère de type polyéther-uréthane.

12. Un conducteur implantable dans le corps tel que revendiqué dans n'importe quelle revendication précédente, comprenant un élément conducteur enroulé disposé à l'intérieur de l'élément conducteur enroulé mentionné en premier; un isolant sur l'élément conducteur enroulé intérieur; l'extrémité distale de l'élément conducteur enroulé extérieur étant connectée à une électrode de pointe (16) et l'extrémité proximale étant connectée à une broche de borne; ce qui forme un conducteur bipolaire dans lequel l'élément conducteur enroulé mentionné en premier est l'élément conducteur enroulé extérieur.

13. Un procédé d'emmanchement d'une électrode annulaire sur un élément conducteur enroulé (38) d'un conducteur implantable dans le corps, comprenant les opérations suivantes:

a. on positionne de façon adjacente à l'extrémité distale de l'élément conducteur enroulé l'électrode annulaire (10), comprenant un chambrage intérieur annulaire (22) pour le dégagement de l'élément conducteur enroulé, à une extrémité distale de l'électrode annulaire, un crochet annulaire (24) à l'extrémité proximale de l'électrode annulaire et deux chambrages annulaires allongés (26, 28) sur le diamètre externe des extrémités distale et proximale de l'électrode annulaire;

b. on engage des bandes d'isolant distale et proximale (30a, 30b) dans les chambrages allongés;

c. on introduit dans l'extrémité distale de l'élément conducteur enroulé un noyau d'emmanchement annulaire (32) comportant une extrémité proximale effilée (34) et une extrémité distale annulaire à crochet (36); et

d. on tire l'électrode annulaire sur le noyau d'emmanchement, ce qui emmanche entre eux l'élément conducteur enroulé, et on fixe l'isolant (12) de l'élément conducteur enroulé à la bande proximale et à la bande distale.

14. Le procédé de la revendication 13, dans lequel l'opération de fixation de l'isolant aux bandes, sur l'électrode annulaire, conduit à l'établissement d'un isolant sur ces bandes, et l'isolant qui recouvre les extrémités de l'électrode annulaire a pratiquement le même diamètre extérieur que l'électrode annulaire.

FIG.1

FIG.2